# EUROPÄISCHE PATENTSCHRIFT

(11) **EP 0 709 464 B1**
(45) Veröffentlichungstag und Bekanntmachung des Hinweises auf die Patenterteilung: **02.03.2005**
(21) Anmeldenummer: 95115277.6
(22) Anmeldetag: 28.09.1995
(51) Int. Cl.: C12P 41/00

(54) **Verfahren zur enzymatischen Enantiomeren-Trennung von rac-2-Oxotricyclo 2,2,1,03,5 -heptan-7-carbonsäure bzw. der -carbonsäureester**
Process for the enzymatic separation of enantiomers of racemic 2-oxotricyclo-2,2,1,03,5-heptan-7-carboxylic acids and esters thereof
Procédé pour la séparation enzymatique des énantiomère d'un mélange racémique d'acide 2-oxotricyclo-2,2,1,03,5-heptan-7-carboxylique et de ses esters

(30) Priorität: 04.10.1994 DE 4435242
(43) Veröffentlichungstag der Anmeldung: 01.05.1996
(73) Patentinhaber: Aventis Pharma Deutschland GmbH, 65929 Frankfurt am Main (DE)
(72) Erfinder: Holla, Wolfgang, Dr., D-65779 Kelkheim (DE); Beck, Gerhard, Dr., D-60320 Frankfurt (DE)

(56) Entgegenhaltungen:
- WO-A-88/03570
- US-A- 5 270 206
- JOURNAL OF THE AMERICAN CHEMICAL SOCIETY, Bd. 95, 1973, Seiten 7522-7523, XP000616106 BINDRA, J.S. ET AL.: "New Extensions of the Bicyclo(2.2.1)heptane Route to Prostaglandins"
- COLLECTION CZECHOSLOVAK CHEM. COMMUN., Bd. 48, 1983, Seiten 3565-3566, XP000616114 CERVINKA O. AND E. HABARTOVA: "Absolute configuration of (1R,2S,4R,6S,7R)-(+)-3-oxo- tricyclo-(2,2,2,02,6)-heptane-7-carboxylic acid"

## Beschreibung

Die Erfindung betrifft ein Verfahren zur Herstellung optisch reiner Verbindungen der Formel I durch stereodifferenzierende Reaktionen der Enantiomeren-Gemische mit Hilfe einer Lipase, Esterase oder Protease.

rac-2-Oxotricyclo[2,2,1,0^{3,5}]heptan-7-carbonsäure (Verbindung der Formel I mit R¹ = H) ist ausgehend von Norbornadien durch Prins-Reaktion und anschließende Jones-Oxidation zugänglich und stellt eine wichtige Vorstufe zur Herstellung von Prostaglandinen dar (Bindra, Grodski, Schaaf, Corey, J. Am. Chem. Soc. 1973, 95, 7522; Cervinka, Habartova, Coll. Czech. Chem. Comm. 1983, 48, 3565; Bartmann, Beck, Jähne, Lerch, Wess, Liebigs Ann. Chem. 1987, 321; CS 240,862).

Es ist seit über zwanzig Jahren bekannt, daß die Racematspaltung von rac-2-Oxotricyclo[2,2,1,0^{3,5}]heptan-7-carbonsäure mit Hilfe von (S)-(-)-Phenylethylamin, durch Herstellung und mehrfaches Umkristallisieren des Phenethylammonium-Salzes, erfolgen kann (Bindra, Grodski, Schaaf, Corey, J. Am. Chem. Soc. 1973, 95, 7522; Bartmann, Beck, Jähne, Lerch, Wess, Liebigs Ann. Chem. 1987, 321; CS 240,862). Dieses Verfahren ist sehr aufwendig und im technischen Maßstab aus ökonomischen Gründen nicht akzeptabel.

Es wurde nun gefunden, daß Verbindungen der Formel I aus den Enantiomeren-Gemischen durch enzymatische Esterspaltung (Hydrolyse) in optisch reiner Form erhalten werden können.

Die kinetische Racematspaltung erfolgt indem man die chiralen, racemischen Carbonsäureester der Formel I in homogenen oder heterogenen wäßrigorganischen Medien einer Lipase-, Esterase- oder Protease-katalysierten stereoselektiven Hydrolyse unterwirft.

Nach Ablauf der Reaktion werden der nicht umgesetzte Ester und die gebildete Säure - und somit beide Enantiomere - getrennt.

Das erfindungsgemäße Verfahren weist insbesondere im technischen Maßstab gegenüber den beschriebenen Verfahren der Racematspaltung mit optisch reinen Aminen (s.o.) folgende Vorteile auf:
- die Durchführung der enzymatischen Enantiomeren-Trennung bzw. Racematspaltung ist ökonomisch, einfach und schnell: sie erfordert keine äquimolaren Mengen optisch reiner Hilfsreagenzien, keine unverhältnismäßig großen Lösungsmittelmengen und auf mehrfaches, aufwendiges, arbeitszeitund geräteintensives Umkristallisieren kann verzichtet werden,
- nach Beendigung der Reaktion kann die Trennung der Produkte durch einfache Maßnahmen, z.B. Extraktion, erfolgen.

Die Erfindung betrifft somit ein Verfahren zur enzymatischen Enantiomeren-Trennung bzw. Racematspaltung, wobei man Enantiomeren-Gemische bzw. racemische Mischungen von Verbindungen der Formel I, in der 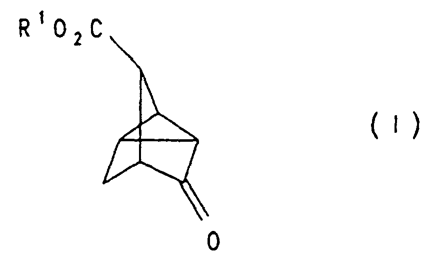
- R¹: C₁-C₁₀-Alkyl, C₃-C₁₀-Cycloalkyl, C₂-C₁₀-Alkenyl, C₂-C₁₀-Alkinyl, C₃-C₈-Cycloalkenyl, C₅-C₁₀-Aryl, C₅-C₁₀-Heteroaryl bedeutet, wobei die Alkyl-, Cycloalkyl-, Alkenyl-, Alkinyl- und Cycloalkenylreste gegebenenfalls einfach, zweifach oder dreifach mit F, Cl, Br, J, CN, CO₂(C₁-C₄)-Alkyl, CONH₂, NR²R³,NO₂, (C₁-C₄)-Alkoxy, O(CO)(C₁-C₄)-Alkoxy, SPh, SMe, SEt, SO₂Ph, SO₂(C₁-C₄)-Alkyl, Aryl, Heteroaryl substituiert sein können,
R², R³ Wasserstoff, C₁-C₆-Alkyl, C₃-C₁₀-Cycloalkyl, C₂-C₆-Alkenyl, C₂-C₆-Alkinyl, Phenyl, Benzyl, Benzoyl oder Aminoschutzgruppen bedeuten oder in der
- R¹: einen Rest der Formel II darstellt, 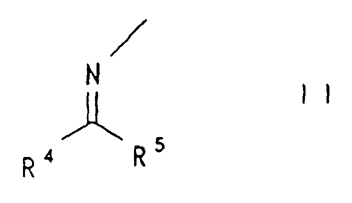 wobei R⁴ und R⁵ Wasserstoff, (C₁-C₆)-Alkyl und R⁴ und R⁵ zusammen einen Alkylen- oder einen Alkenylen-Rest darstellen
oder in der
- R¹: einen Rest der Formel III darstellt, 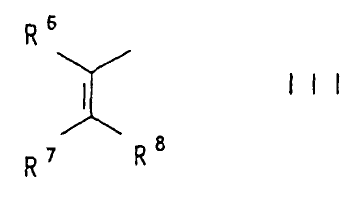 wobei R⁶, R⁷ und R⁸ Wasserstoff, Methyl oder Ethyl darstellt, oder in der
- R¹: einen Rest der Formel IV darstellt, 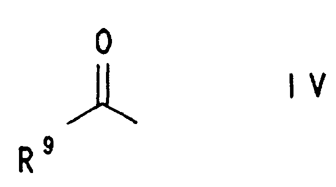 wobei R⁹ Wasserstoff, (C₁-C₄)-Alkyl oder 2-Oxotricyclo[2,2,1,0^{3,5}]-heptan-7-carbonyl bedeutet,
in Gegenwart von Lipasen und Esterasen aus Schweineleber, Schweinepankreas, Rinderpankreas oder aus Mikroorganismen wie Candida, Pseudomonas, Mucor, Rhizopus und Aspergillus oder Proteasen aus Bacillus in Gegenwart von Wasser und gegebenenfalls einem Cosolvens umsetzt und die in der erhaltenen Lösung vorliegenden Ester und Säuren voneinander trennt.

Die oben und nachfolgend genannten Alkyl, Alkenyl und Alkinylgruppen können sowohl geradkettig als auch verzweigt sein.

Unter dem Begriff Aryl sollen aromatische Verbindungen mit 6 bis 12 C-Atomen verstanden werden, beispielsweise Phenyl oder Naphthyl.

Unter dem Begriff Heteroaryl sollen aromatische Verbindungen mit 3 bis 7 C-Atomen verstanden werden, die mindestens ein N-, O- oder S-Atom enthalten, beispielsweise Furyl oder Thienyl.

Unter dem Begriff Aminoschutzgruppe sind allgemein gebräuchliche Aminoschutzgruppen zu verstehen, beispielsweise Benzyloxycarbonyl (CBz = Z), Butyloxycarbonyl (BOC), Allyloxycarbonyl (ALOC) oder Acetyl (Ac).

Bevorzugt werden racemische Mischungen von Verbindungen der Formel I eingesetzt, in denen
- R¹: C₁-C₁₀-Alkyl, C₂-C₁₀-Alkenyl, bedeutet, wobei die Alkylreste gegebenenfalls (auch mehrfach) mit F, CL, Br, J, CN, CO₂(C₁-C₄)-Alkyl, NO₂, (C₁-C₄)-Alkoxy und O(CO)(C₁-C₄)-Alkoxy, substituiert sein können,
oder in denen
- R¹: einen Rest der Formel II darstellt, wobei R⁴ und R⁵ Wasserstoff oder Methyl bedeuten
oder in der
- R¹: einen Rest der Formel III darstellt, wobei R⁶, R⁷ und R⁸ Wasserstoff oder Ethyl darstellen,
oder in der
- R¹: einen Rest der Formel IV darstellt, wobei R⁹ Wasserstoff, (C₁-C₄)-Alkyl oder 2-Oxotricyclo[2,2,1,0^{3,5}]heptan-7-carbonyl bedeutet.

Besonders bevorzugt sind Verbindungen der Formel I, in denen
- R¹: Methyl oder Ethyl bedeutet
oder in denen
- R¹: einen Rest der Formel II darstellt, wobei R⁴ und R⁵ Methyl darstellen,
oder in denen
- R¹: einen Rest der Formel III darstellt, wobei R⁶, R⁷ und R⁸ Wasserstoff darstellen.

Bei dem erfindungsgemäßen Verfahren geht man vorzugsweise so vor, daß man einen Ester der Formel I, beispielsweise den Methyl- (R¹ = CH₃) oder den Vinylester (R¹ = Rest der Formel III mit R⁶ = R⁷ = R⁸ = H), in einer wäßrigen Lösung mit einer Lipase, Esterase oder Protease versetzt und rührt. Es kann vorteilhaft sein, die genannte Lösung zu puffern, z.B. mit Phthalat-, Phosphat-, TRIS[ = Tris-(hydroxymethyl)-methylamin]-Puffer. Der Zusatz an Puffer kann z.B 0.01 -1.0 M sein; ein günstiger Pufferbereich ist pH 5-9. Weiterhin kann es vorteilhaft sein, der Reaktionslösung ein organisches Lösungsmittel als Cosolvens zuzusetzen. Als Cosolvens eignen sich z.B. Dimethoxyethan, Aceton, THF, Dioxan, Hexan und tert.-Butylmethylether. Der Anteil an Cosolvens in der Lösung liegt vorzugsweise bei 10 bis 70 %.

Als Enzyme werden bevorzugt die Lipase SP 526 aus Candida antarctica (käuflich bei Novo Nordisk), die Schweineleber-Esterase (PLE, käuflich bei Sigma Chemical Co.), die Lipase OF aus Candida cylindracea (käuflich bei Meito Sangyo Co.) und die Lipase P (oder FP) aus Pseudomonas spec. (käuflich bei Amano Pharmaceuticals, Nagoya, Japan) sowie die Alcalase (Protease aus Bacillus licheniformis, käuflich bei Novo Nordisk) verwendet. Alternativ lassen sich die genannten Enzyme nach Methoden des Standes der Technik herstellen: Methoden zur Züchtung - ggf. unter Einbeziehung gentechnischer Methoden - der genannten Mikroorganismen, Verfahren zur Charakterisierung, Produktion und Reinigung der gewünschten Enzyme finden sich in: Mikrobiologische und Biochemische Verfahrenstechnik, A. Einsele et al. VCH Weinheim, 1985; Methods of Enzymatic Analysis, 3rd Edition, Vol. I-XII, H.-U. Bergmeyer, VCH Publishers, Weinheim 1986; Enzymes, 3rd Edition, M.Dixon, E.C. Webb, Academic Press, Inc. 1979.

Jedes der genannten Enzyme kann in freier oder in immobilisierter Form (Immobilized Biocatalysts, W. Hartmeier, Springer Verlag Berlin, 1988) eingesetzt werden. Die Enzymmenge wird in Abhängigkeit von der Reaktionsgeschwindigkeit bzw. von der angestrebten Reaktionszeit (bzw. dem angestrebten Umsatz) und von der Art des Enzyms (z.B. frei oder immobilisiert) frei gewählt und ist durch einfache Vorversuche leicht zu bestimmen. Die Reaktionsmischung enthält vorzugsweise 2-50 Gew.-% Ester, besonders bevorzugt 5-20 % Ester. Die Reaktionstemperatur beträgt 10-100°C, bevorzugt 25-80°C.

Die Überführung von rac-2-Oxotricyclo[2,2,1,0^{3,5}]heptan-7-carbonsäure (Verbindung der Formel I mit R¹ = H) in die entsprechenden Ester (Verbindung der Formel I) kann mit bekannten chemischen Methoden der Veresterung erfolgen (Haslam, Tetrahedron 1980, 36, 2409; Höfle, Steglich, Vorbrüggen, Angew. Chem. 1978, 90, 602; Geckeler, Bayer, Chem. Ber. 1974, 107, 1271).

Die bei dem Verfahren entstehenden bzw. verbleibenden Produkte lassen sich auf einfache Weise trennen, z.B. durch Ausfällen, chromatographische Methoden oder Extraktion, vorzugsweise Extraktion. Den Ester erhält man z.B. durch einfache Extraktion der wäßrigen Reaktionsmischung, z.B. mit tert.-Butylmethylether; die durch Hydrolyse entstandene Säure erhält man z.B. durch Einengen (z.B. Gefriertrocknung) der Wasserphase.
Abtrennung (und spätere Wiederverwendung) des Enzyms werden durch eine Immobilisierung erleichtert.

Durch geeignete Reaktionsführung gelingt es zumindest immer ein Enantiomeres optisch rein zu erhalten. Strebt man optisch reinen Ester an, sollte der Umsatz über 50 % liegen, steht optisch reine Säure im Vordergrund des Interesses, sollte der Umsatz kleiner 50 % sein.

Die Umsatzbestimmung der enzymatischen Hydrolyse erfolgte mit HPLC (RP 8 LiChrosper® 60), die Bestimmung der optischen Reinheit wurde mit HPLC (Chiralcel® OD), GC (FS Lipodex^{R}) und durch Vergleich der Drehwerte durchgeführt. Durch die nachfolgenden Beispiele sowie durch den Inhalt der Patentansprüche soll die vorliegende Erfindung näher erläutert werden.

### Beispiel 1

2.0 g (0.012 mol) rac-2-Oxotricyclo[2.2.1.0^{3,5}]heptan-7-carbonsäuremethylester (Verbindung der Formel I mit R¹ = Me) werden in 50 ml 0.1 M Kaliumphosphat-Puffer, pH = 7, bei 23°C mit 300 mg Lipase SP 526 aus Candida antarctica (Novo Nordisk) mit dem Magnetrührer gerührt. Der pH-Wert wird mit Hilfe eines Autotitrators konstant gehalten. Nach Zudosieren von etwa 14 ml 0.5 NaOH, wird das Reaktionsgemisch mit tert.-Butylmethylether extrahiert. Nach Trocknen mit Na₂SO₄ und Einengen der organischen Phase erhält man 0.81 g (0.0049 mol, 82 %) optisch reinen (HPLC [Chiracel OD, n-Hexan + EtOH:100+1,1 ml/min, 210 nm]; 100 % ee) (1R,7R)-(+)-2-Oxotricyclo[2.2.1.0^{3,5}]heptan-7-carbonsäuremethylester.

### Beispiel 2

2.0 g (0.012 mol) rac-2-Oxotricyclo[2.2.1.0^{3,5}]heptan-7-carbonsäuremethylester (Verbindung der Formel I mit R¹ = Me) werden in einer Mischung aus 25 ml 0.05 M Kaliumphosphat-Puffer, pH = 7, und 5 ml Aceton bei 40°C mit 300 mg Lipase SP 526 aus Candida antarctica (Novo Nordisk) mit dem Magnetrührer gerührt. Der pH-Wert wird mit Hilfe eines Autotitrators konstant gehalten. Nach Zudosieren von etwa 14.4 ml 0.5 M NaOH, wird das Reaktionsgemisch mit tert.-Butylmethylether extrahiert. Nach Trocknen mit Na₂SO₄ und Einengen der organischen Phase erhält man 0.80 g (0.0048 mol, 80 %) optisch reinen (HPLC [Chiracel OD, n-Hexan + EtOH:100+1,1 ml/min, 210 nm]; 100 % ee) (1R,7R)-(+)-2-Oxotricyclo[2.2.1.0^{3,5}]heptan-7-carbonsäuremethylester.

### Beispiel 3

2.0 g (0.011 mol) rac-2-Oxotricyclo[2.2.1.0^{3,5}]heptan-7-carbonsäureethylester (Verbindung der Formel I mit R¹ = Et) werden in 25 ml 0.1 M Kaliumphosphat-Puffer, pH = 7, bei 50°C mit 300 mg Lipase SP 526 aus Candida antarctica (Novo Nordisk) mit dem Magnetrührer gerührt. Der pH-Wert wird mit Hilfe eines Autotitrators konstant gehalten. Nach Zudosieren von etwa 12.0 ml 0.5 M NaOH, wird das Reaktionsgemisch mit tert.-Butylmethylether extrahiert. Nach Trocknen mit Na₂SO₄ und Einengen der organischen Phase erhält man 0.89 g (0.005 mol, 90 %) (1R,7R)-(+)-2-Oxotricyclo[2.2.1.0^{3,5}]heptan-7-carbonsäureethylester (HPLC [Chiracel OD,
n-Hexan + EtOH:100+1,1 ml/min, 210 nm]; 98 % ee).

### Beispiel 4

230.0 g (1.386 mol) rac-2-Oxotricyclo[2.2.1.0^{3,5}]heptan-7-carbonsäuremethylester (Verbindung der Formel I mit R¹ = Me) werden in einer Mischung aus 2.7 l 0.05 M Kaliumphosphat-Puffer, pH = 7, und 300 ml Dimethoxyethan bei 50°C mit 34.5 g Lipase SP 526 aus Candida antarctica (Novo Nordisk) gerührt. Der pH-Wert wird mit Hilfe eines Autotitrators konstant gehalten. Nach Zudosieren von etwa 750 ml 1.0 M NaOH, wird das Reaktionsgemisch mit tert.-Butylmethylether extrahiert. Nach Trocknen mit Na₂SO₄ und Einengen der organischen Phase erhält man 88.6 g (0.536 mol, 77.4%) (1 1R,7R)-(+)-2-Oxotricyclo[2.2.1.0^{3,5}]heptan-7-carbonsäuremethylester (GC [FS Lipodex E, 120°C, H₂, t_{Ret}: 46.7 min]: > 99 % ee).

### Beispiel 5

50 mg (0.28 mmol) rac-2-Oxotricyclo[2.2.1.0^{3,5}]heptan-7-carbonsäurevinylester (Verbindung der Formel I mit R¹ = Rest der Formel III mit R⁶ = R⁷ = R⁸ = H) werden in einer Mischung aus 2 ml 1.0 M Kaliumphosphat-Puffer, pH = 7, bei 23°C mit 10 mg Lipase OF aus Candida cylindracea (Meito Sangyo) mit dem Magnetrührer gerührt. Nach 6 h wird das Reaktionsgemisch mit tert.-Butylmethylether extrahiert. Nach Trocknen mit Na₂SO₄ und Einengen der organischen Phase erhält man 15 mg (0.084 mmol, 60 %) (1R,7R)-(+)-2-Oxotricyclo[2.2.1.0^{3,5}]heptan-7-carbonsäurevinylester (HPLC [Chiracel OD, n-Hexan+EtOH:100+1,1 mL/min, 210 nm}: 95 % ee).

## Patentansprüche

1. Verfahren zur enzymatischen Enantiomeren-Trennung bzw. Racematspaltung, von Enantiomeren-Gemischen bzw. racemischen Mischungen von Verbindungen der Formel I, in der 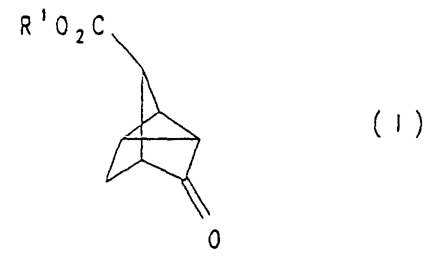
R¹ C₁-C₁₀-Alkyl, C₃-C₁₀-Cycloalkyl, C₂-C₁₀-Alkenyl, C₂-C₁₀-Alkinyl, C₃-C₈-Cycloalkenyl, C₅-C₁₀-Aryl, C₅-C₁₀-Heteroaryl bedeutet, wobei die Alkyl-, Cycloalkyl-, Alkenyl-, Alkinyl- und Cycloalkenylreste gegebenenfalls einfach, zweifach oder dreifach mit F, Cl, Br, J, CN, CO₂(C₁-C₄)-Alkyl, CONH₂, NR²R³,NO₂, (C₁-C₄)-Alkoxy, O(CO)(C₁-C₄)-Alkoxy, SPh, SMe, SEt, SO₂Ph, SO₂(C₁-C₄)-Alkyl, Aryl, Heteroaryl substituiert sein können,
R², R³ Wasserstoff, C₁-C₆-Alkyl, C₃-C₁₀-Cycloalkyl, C₂-C₆-Alkenyl, C₂-C₆-Alkinyl, Phenyl, Benzyl, Benzoyl oder Aminoschutzgruppen bedeuten oder in der
R¹ einen Rest der Formel II darstellt, 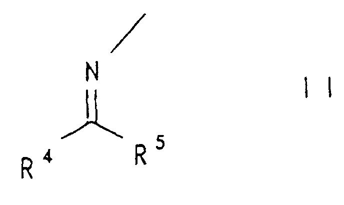 wobei R⁴ und R⁵ Wasserstoff, (C₁-C₆)-Alkyl und R⁴ und R⁵ zusammen einen Alkylen- oder einen Alkenylen-Rest darstellen
oder in der
R¹ einen Rest der Formel III darstellt, 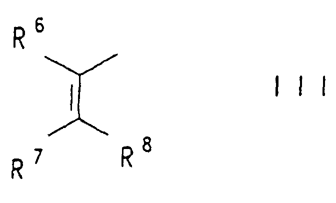 wobei R⁶, R⁷ und R⁸ Wasserstoff, Methyl oder Ethyl darstellt, oder in der
R¹ einen Rest der Formel IV darstellt, 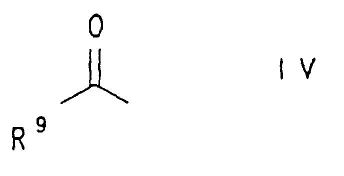 wobei R⁹ Wasserstoff, (C₁-C₄)-Alkyl oder 2-Oxotricyclo[2,2,1,0^{3,5}]-heptan-7-carbonyl bedeutet,
**dadurch gekennzeichnet, daß** man die Verbindung der Formel I
in Gegenwart einer Lipase oder Esterase aus Schweineleber, Schweinepankreas, Rinderpankreas oder aus Mikroorganismen wie Candida, Pseudomonas, Mucor, Rhizopus und Aspergillus oder Proteasen aus Bacillus in Gegenwart von Wasser und gegebenenfalls einem Cosolvens umsetzt und die in der erhaltenen Lösung vorliegenden Ester und Säuren voneinander trennt.

2. Verfahren gemäß Anspruch 1, **dadurch gekennzeichnet, daß** von den Substituenten der Formel I
R¹ C₁-C₁₀-Alkyl, C₂-C₁₀-Alkenyl, bedeutet, wobei die Alkylreste gegebenenfalls (auch mehrfach) mit F, CL, Br, J, CN, CO₂(C₁-C₄)-Alkyl, NO₂, (C₁-C₄)-Alkoxy und O(CO)(C₁-C₄)-Alkoxy, substituiert sein können,
oder
R¹ einen Rest der Formel II darstellt, wobei R⁴ und R⁵ Wasserstoff oder Methyl bedeuten
oder
R¹ einen Rest der Formel III darstellt, wobei R⁶, R⁷ und R⁸ Wasserstoff oder Ethyl darstellen,
oder
R¹ einen Rest der Formel IV darstellt, wobei R⁹ Wasserstoff, (C₁-C₄)-Alkyl oder 2-Oxotricyclo[2,2,1,0^{3,5}]heptan-7-carbonyl bedeutet.

3. Verfahren gemäß Anspruch 1 oder 2, **dadurch gekennzeichnet, daß** von den Substituenten der Formel I
R¹ Methyl oder Ethyl bedeutet
oder
R¹ einen Rest der Formel II darstellt, wobei R⁴ und R⁵ Methyl darstellen,
oder
R¹ einen Rest der Formel III darstellt, wobei R⁶, R⁷ und R⁸ Wasserstoff darstellen.

4. Verfahren gemäß einem oder mehrern der Ansprüche 1-3, **dadurch gekennzeichnet, daß** eine Lipase eingesetzt wird, die aus Candida antarctica gewonnen wurde.

5. Verfahren gemäß einem oder mehreren der Ansprüche 1-4, **dadurch gekennzeichnet, daß** das Enzym in immobilisierter Form eingesetzt wird.

6. Verfahren gemäß einem oder mehreren der Ansprüche 1-5, **dadurch gekennzeichnet, daß** die Reaktionsmischung 2-50 % des Esters der Formel I enthält.

## Claims

1. A process for the enzymatic separation of enantiomers or resolution of enantiomeric mixtures or racemic mixtures of compounds of the formula I 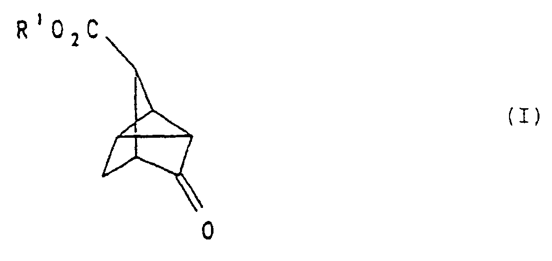 in which
R¹ is C₁-C₁₀-alkyl, C₃-C₁₀-cycloalkyl, C₂-C₁₀-alkenyl, C₂-C₁₀-alkynyl, C₃-C₈-cycloalkenyl, C₅-C₁₀-aryl or C₅-C₁₀-heteroaryl, it optionally being possible for the alkyl, cycloalkyl, alkenyl, alkynyl and cycloalkenyl radicals to be monosubstituted, disubstituted or trisubstituted by F, Cl, Br, I, CN, CO₂(C₁-C₄)-alkyl, CONH₂, NR²R³, NO₂, (C₁-C₄)-alkoxy, O(CO) (C₁-C₄)-alkoxy, SPh, SMe, SEt, SO₂Ph, SO₂(C₁-C₄)-alkyl, aryl or heteroaryl,
R² and R³ are hydrogen, C₁-C₆-alkyl, C₃-C₁₀-cycloalkyl, C₂-C₆-alkenyl, C₂-C₆-alkynyl, phenyl, benzyl, benzoyl or amino protective groups, or in which
R¹ is a radical of the formula II 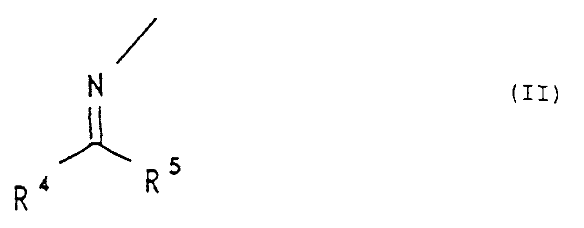
R⁴ and R⁵ being hydrogen or (C₁-C₆)-alkyl and R⁴ and R⁵ together being an alkylene or an alkenylene radical
or in which
R¹ is a radical of the formula III 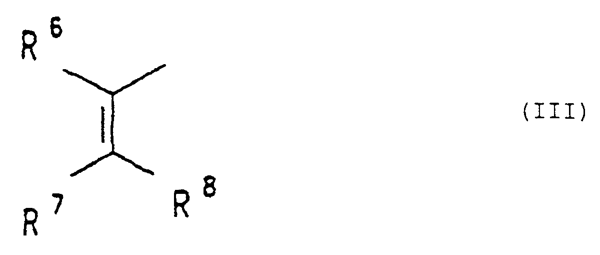
R⁶, R⁷ and R⁸ being hydrogen, methyl or ethyl,
or in which
R¹ is a radical of the formula IV 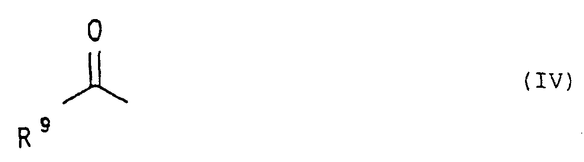
R⁹ being hydrogen, (C₁-C₄)-alkyl or 2-oxotricyclo-[2.2.1.0^{3,5}]heptane-7-carbonyl,
which comprises reacting the compound of the formula I in the presence of a lipase or esterase from porcine liver, porcine pancreas, bovine pancreas or from microorganisms such as Candida, Pseudomonas, Mucor, Rhizopus and Aspergillus, or proteases from Bacillus in the presence of water and, if appropriate, a cosolvent and separating from one another the esters and acids present in the solution obtained.

2. The process as claimed in claim 1, wherein, of the substituents of the formula I,
R¹ is C₁-C₁₀-alkyl or C₂-C₁₀-alkenyl, it optionally being possible for the alkyl radicals to be substituted (even several times) by F, Cl, Br, I, CN, CO₂(C₁-C₄)-alkyl, NO₂, (C₁-C₄)-alkoxy and O(CO)(C₁-C₄)-alkoxy,
or
R¹ is a radical of the formula II, R⁴ and R⁵ being hydrogen or methyl
or
R¹ is a radical of the formula III, R⁶, R⁷ and R⁸ being hydrogen or ethyl,
or
R¹ is a radical of the formula IV, R⁹ being hydrogen, (C₁-C₄)-alkyl or 2-oxotricyclo[2.2.1.0^{3,5}]-heptane-7-carbonyl.

3. The process as claimed in claim 1 or 2, wherein, of the substituents of the formula I,
R¹ is methyl or ethyl,
or
R¹ is a radical of the formula II, R⁴ and R⁵ being methyl,
or
R¹ is a radical of the formula III, R⁶, R⁷ and R⁸ being hydrogen.

4. The process as claimed in one or more of claims 1-3, wherein a lipase is employed which was obtained from Candida antarctica.

5. The process as claimed in one or more of claims 1-4, wherein the enzyme is employed in immobilized form.

6. The process as claimed in one or more of claims 1-5, wherein the reaction mixture contains 2-50% of the ester of the formula I.

## Revendications

1. Procédé pour la séparation enzymatique d'énantiomères ou la dissociation enzymatique d'un racémate de mélanges d'énantiomères ou de mélanges racémiques de composés de formule I 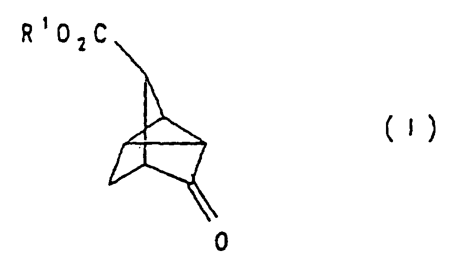 dans laquelle R¹ signifie un radical alkyle en C₁ à C₁₀, cycloalkyle en C₃ à C₁₀, alcényle en C₂ à C₁₀, alcynyle en C₂ à C₁₀, cycloalcényle en C₃ à C₈, aryle en C₅ à C₁₀, hétéroaryle en C₅ à C₁₀, où les radicaux alkyle, cycloalkyle, alcényle, alcynyle et cycloalcényle peuvent le cas échéant être monosubstitués, disubstitués ou trisubstitués par F, Cl, Br, I, CN, CO₂(alkyle en C₁ à C₄), CONH₂, NR²R³, NO₂, alcoxy en C₁ à C₄, O(CO) (alcoxy en C₁ à C₄), SPh, SMe, SEt, SO₂Ph, SO₂(alkyle en C₁ à C₄), aryle, hétéroaryle,
R², R³ signifiant hydrogène, alkyle en C₁ à C₆, cycloalkyle en C₃ à C₁₀, alcényle en C₂ à C₆, alcynyle en C₂ à C₆, phényle, benzyle, benzoyle ou des groupes de protection de la fonction amino ou dans laquelle
R¹ représente un radical de formule II, 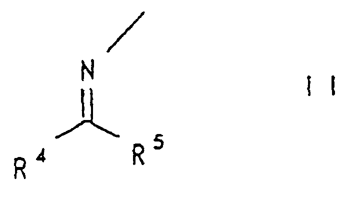 où R⁴ et R⁵ représentent hydrogène, alkyle en C₁ à C₆, ou R⁴ et R⁵ représentent ensemble un radical alkylène ou alcénylène
ou dans laquelle
R¹ représente un radical de formule III, 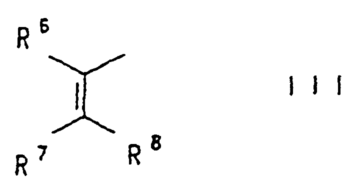 où R⁶, R⁷ et R⁸ représentent hydrogène, méthyle ou éthyle
ou dans laquelle
R¹ représente un radical de formule IV, 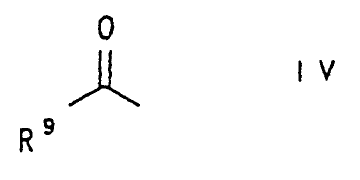 où R⁹ représente hydrogène, alkyle en C₁ à C₄ ou 2-oxotricyclo(2,2,1,0^{3,5})-heptane-7-carbonyle,
**caractérisé en ce qu'**on transforme le composé de formule I en présence d'une lipase ou d'une estérase provenant du foie de porc, du pancréas de porc, du pancréas de boeuf ou de microorganismes tels que Candida, Pseudomonas, Mucor, Rhizopus et Aspergillus ou de protéases provenant de Bacillus en présence d'eau et le cas échéant d'un cosolvant et on sépare les uns des autres les esters et acides se trouvant dans la solution obtenue.

2. Procédé selon la revendication 1, **caractérisé en ce que** parmi les substituants de la formule I
R¹ signifie alkyle en C₁ à C₁₀, alcényle en C₂ à C₁₀, les radicaux alkyle pouvant le cas échéant être substitués (également polysubstitués) par F, Cl, Br, I, CN, CO₂ (alkyle en C₁ à C₄), NO₂, alkyloxy en C₁ à C₄ et O(CO) (alcoxy en C₁ à C₄)
ou
R¹ représente un radical de formule II, où R⁴ et R⁵ signifient hydrogène ou méthyle
ou
R¹ représente un radical de formule III, où R⁶, R⁷ et R⁸ représentent hydrogène ou éthyle
ou
R¹ représente un radical de formule IV, où R⁹ signifie hydrogène, alkyle en C₁ à C₄ ou 2-oxotricyclo[2,2,1,0^{3,5}]heptane-7-carbonyle.

3. Procédé selon la revendication 1 ou 2, **caractérisé en ce que** parmi les substituants de la formule I,
R¹ signifie méthyle ou éthyle
ou
R¹ représente un radical de formule II, où R⁴ et R⁵ représentent méthyle,
ou
R¹ représente un radical de formule III, où R⁶, R⁷ et R⁸ représentent hydrogène.

4. Procédé selon l'une ou plusieurs des revendications 1 à 3, **caractérisé en ce qu'**on utilise une lipase obtenue à partir de Candida antarctica.

5. Procédé selon l'une ou plusieurs des revendications 1 à 4, **caractérisé en ce que** l'enzyme est utilisée sous forme immobilisée.

6. Procédé selon l'une ou plusieurs des revendications 1 à 5, **caractérisé en ce que** le mélange réactionnel contient 2 à 50% de l'ester de formule I.
